# FASCICULE DE BREVET EUROPEEN

(11) **EP 2 180 039 B1**
(45) Date de publication et mention de la délivrance du brevet: **07.10.2015**
(21) Numéro de dépôt: 09305979.8
(22) Date de dépôt: 14.10.2009
(51) Int. Cl.: C12M 1/02

(54) **Réceptacle pour la réception d'échantillon biologique ainsi qu'une cellule d'étude de l'environnement climatique logeant un tel réceptacle**
Behälter zur Aufnahme einer biologischen Probe, und Zelle zu klimatischen Umgebungsstudienzwecken, in der sich dieser Behälter befindet
Container for receiving a biological sample as well as a climatic environment research cell containing such a container

(30) Priorité: 27.10.2008 FR 0805950
(43) Date de publication de la demande: 28.04.2010
(73) Titulaire: JF Cesbron, 49480 Saint-Sylvain-d'Anjou (FR); Centre National de la Recherche Scientifique, 75794 Paris Cedex 16 (FR)
(72) Inventeur: Verdier, Bruno, 77000, MELUN (FR); Rabin, Christian, 72200, LA FLECHE (FR); Simonnet, Benoît, 44300, NANTES (FR)
(74) Mandataire: Laget, Jean-Loup

(56) Documents cités:
- EP-A- 0 614 688
- DE-A1- 4 028 871

## Description

La présente invention concerne un réceptacle pour la réception d'échantillon biologique ainsi qu'une cellule d'étude de l'environnement climatique logeant un tel réceptacle.

De nombreux échantillons biologiques sont prélevés dans des milieux dans lesquels ils sont soumis à des variations de température en fonction de leur profondeur dans ledit milieu. La difficulté est de reproduire sur l'échantillon, une fois prélevé, le différentiel thermique observé dans des conditions naturelles à différentes profondeurs entre un volume (sol ou eau) dont l'échantillon est issu et l'atmosphère ambiante. Or, l'intégration de ce différentiel, par exemple pour l'étude d'écosystème terrestre ou aquatique, est une clé importante de compréhension de la dynamique des systèmes biologiques. Jusqu'à présent, dans le cas par exemple d'étude d'impact du climat sur un écosystème, on se contente de placer l'échantillon de l'écosystème à étudier sur un support, tel qu'une table, elle-même disposée dans l'enceinte d'une cellule climatique avant de soumettre ledit échantillon à des variations de climat par modification de l'atmosphère de l'enceinte. Toutefois, à aucun moment on ne tient compte, lors de cette étude, du niveau d'enfouissement de l'échantillon dans le sol et du différentiel thermique résultant de cet enfouissement. Par ailleurs, la conception du support sous forme d'une simple table empêche toute observation des migrations au sein de l'échantillon lorsqu'il est replacé dans un milieu conforme à son environnement naturel en termes de différentiel thermique.

Il est également connu de l'état de la technique, comme l'illustre DE-40 28 871 une cuve de réaction pour la culture de microorganismes, cette cuve présentant un corps de cuve comportant une double paroi, le volume annulaire formé par la double paroi étant rempli d'un liquide caloporteur qui régule la température de ladite cuve de réaction.

Par ailleurs, le document EP-0.614.688 décrit un réacteur de cristallisation à double paroi dont le volume annulaire est divisé en plusieurs zones.

Un but de la présente invention est de proposer un réceptacle pour échantillon biologique dont la conception permet de simuler les gradients de température naturels observés par exemple dans les écosystèmes terrestres ou aquatiques et d'observer en parallèle les migrations au sein d'un tel échantillon replacé dans des conditions de température conformes à son milieu naturel.

A cet effet, l'invention a pour objet un réceptacle pour la réception d'échantillon biologique, **caractérisé en ce que** ledit réceptacle est formé d'une cuve cylindrique de réception d'échantillon, isolée thermiquement de l'extérieur, et d'un socle sur lequel la cuve est, en position dressée dite verticale, apte à reposer, ledit socle étant équipé d'au moins trois capteurs de poids à lecture indépendante, lesdits capteurs de poids répartis à la surface du socle étant sollicités en position d'appui de la cuve sur le socle, cette cuve présentant un corps de cuve comportant une double paroi divisée en au moins deux zones décalées le long de l'axe longitudinal de la cuve, chaque zone, qui délimite un volume annulaire régulable en température à l'aide de moyens de chauffage et de refroidissement, formant un échangeur thermique apte à refroidir ou à réchauffer le contenu de la cuve, chaque échangeur étant isolé thermiquement du ou des autres échangeur(s) thermique(s).

La réalisation de la cuve sous forme d'une superposition d'échangeurs thermiques à fonctionnement indépendant les uns des autres permet de simuler le différentiel thermique observé dans le milieu naturel de l'échantillon tandis que la présence des capteurs, qui délivrent chacun une information, permet d'étudier les migrations se produisant dans un tel échantillon replacé dans des conditions de température conformes à son milieu naturel.

De préférence, le socle est équipé de moyens de guidage et de centrage de la cuve lors du positionnement de la cuve sur ledit socle.

Ces moyens de guidage et de centrage permettent de garantir un fonctionnement optimal des capteurs dont la position relative par rapport au pourtour de la cuve est parfaitement connue.

De préférence, au moins l'un des échangeurs thermiques constitutifs du corps de cuve, dit échangeur supérieur, se présente sous forme d'une pièce du type couronne séparable du reste du corps de cuve pour la réalisation d'une cuve de hauteur ajustable.

La réalisation d'au moins l'un des échangeurs thermiques sous forme d'une pièce amovible permet de faire varier la hauteur de la cuve en positionnant ou non ladite pièce sur le corps de cuve. La réduction de la hauteur de la cuve permet, dans le cas d'un placement du réceptacle dans une cellule de l'environnement climatique, de faire plus aisément varier d'autres paramètres, tels que la hauteur d'éclairage du contenu de la cuve, la force des précipitations, etc.

Pour parfaire la simulation d'un milieu naturel, la cuve présente un fond à double paroi délimitant entre lesdites parois un volume régulable en température à l'aide de moyens de chauffage et de refroidissement dudit volume, ce fond formant un échangeur thermique supplémentaire apte à réchauffer ou à refroidir le contenu de la cuve.

De préférence, la cuve étant montée amovible sur le socle, le fond de la cuve est muni d'une embase équipée de passages de fourche pour la manutention de la cuve.

Ainsi, la cuve peut être aisément manipulée à l'aide d'un chariot à fourche traditionnel et peut être amenée sur le site de prélèvement de l'échantillon.

De préférence, ladite embase est équipée de pieds ou semelles d'appui au sol de la cuve, lesdits pieds ou semelles étant disposés à l'aplomb des capteurs de poids à l'état posé de la cuve sur son socle. Ces pieds ou semelles évitent tout endommagement du fond de la cuve lorsque celle-ci repose directement au sol.

De préférence, chaque échangeur thermique est muni de moyens de raccordement à un réseau d'alimentation en fluide régulable en température, ce fluide formant les moyens de chauffage et de refroidissement dudit échangeur, lesdits moyens de raccordement étant formés au moins par une entrée et une sortie de fluide à raccords rapides ménagées dans la paroi périphérique externe isolée thermiquement de la cuve à double paroi

L'utilisation, à titre de moyens de chauffage ou de refroidissement, d'un fluide permet une régulation aisée et rapide de la température à un faible coût.

Généralement, les informations issues des capteurs de poids sont stockées à l'intérieur d'un système électronique et/ou informatique de commande, comportant des moyens de pilotage des moyens de chauffage et de refroidissement des échangeurs thermiques.

L'invention a encore pour objet une cellule d'étude de l'environnement climatique apte à recevoir un échantillon biologique à étudier, ladite cellule comprenant au moins :
- une enceinte,
- des moyens de génération de l'environnement climatique aptes à modifier l'état de l'atmosphère intérieur de l'enceinte de ladite cellule, cet état se caractérisant au moins par les paramètres température, humidité, ensoleillement, pression, vent, précipitations, teneur en gaz et
- un réceptacle logé à l'intérieur de l'enceinte de ladite cellule,
**caractérisée en ce que** le réceptacle servant à la réception de l'échantillon est du type précité.

L'invention sera bien comprise à la lecture de la description suivante d'exemples de réalisation, en référence aux dessins annexés dans lesquels :

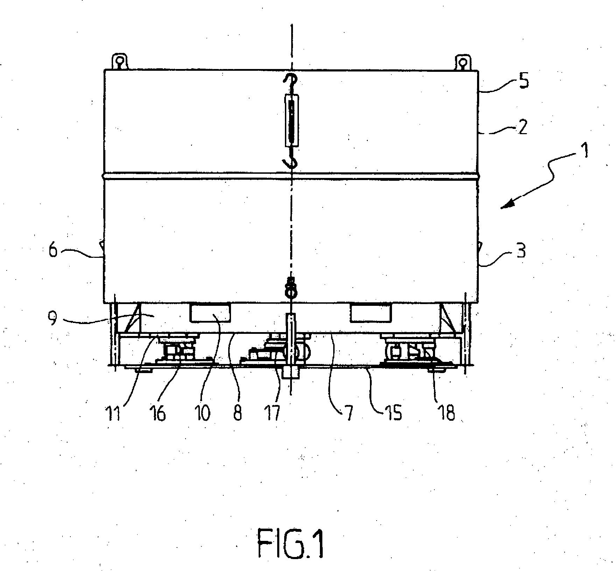

la figure 1 représente une vue en perspective d'un réceptacle à l'état posé de la cuve sur le socle ;

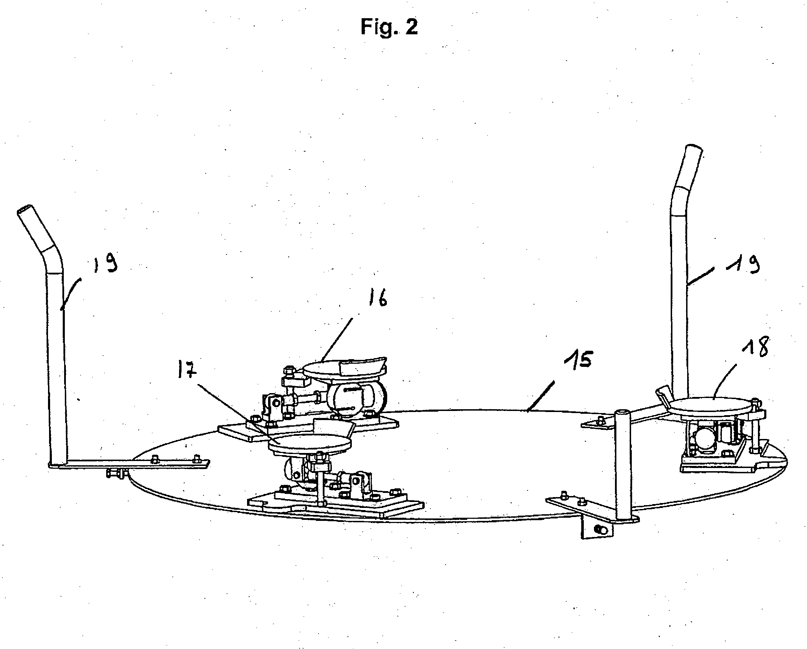

la figure 2 représente une vue en perspective du socle seul ;

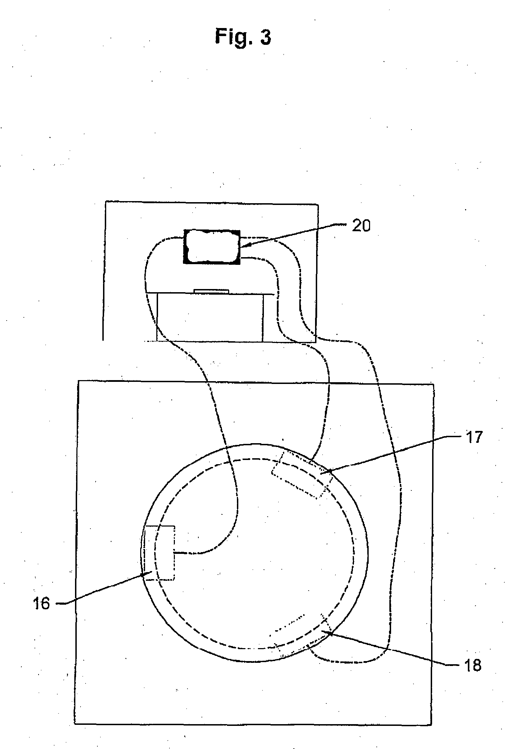

la figure 3 représente une vue schématique de dessus d'un réceptacle et de son système électronique et/ou informatique de commande et
la figure 4 représente une vue en coupe de la cuve illustrant les raccordements des échangeurs thermiques constitutifs de la cuve à un réseau d'alimentation en fluide régulé en température.

Comme mentionné ci-dessus, le réceptacle, objet de l'invention, est plus particulièrement destiné à l'étude d'échantillon biologique, c'est-à-dire incluant au moins un être vivant. Ce réceptacle présente l'avantage de pouvoir, du fait de sa conception, simuler les différentiels thermiques auxquels l'échantillon est soumis dans des conditions naturelles et étudier en parallèle les migrations se produisant au sein dudit échantillon. Bien évidemment, un tel réceptacle permet également de simuler des variations de ce différentiel thermique et d'en observer les conséquences sur l'échantillon dans des conditions déterminées, notamment lorsque le réceptacle est placé dans une cellule d'étude de l'environnement climatique équipée de moyens de génération de climat.

Le réceptacle 1, objet de l'invention, est donc formé d'une cuve 2 cylindrique de réception d'échantillon isolée thermiquement de l'extérieur et d'un socle 15 sur lequel la cuve 2 repose, en position dressée dite verticale.

Dans les exemples représentés, le socle 15 est équipé d'au moins trois capteurs 16, 17, 18 de poids à lecture indépendante. En conséquence, chaque capteur délivre une information qui peut être traitée indépendamment des informations délivrées par les autres capteurs. Les capteurs 16, 17, 18 de poids sont répartis à la surface du socle 15 et sont sollicités lorsque la cuve 2 est disposée en position d'appui sur le socle 15. Ce socle 15 est formé ici par une platine circulaire. Les capteurs 16, 17, 18 de poids sont en disposition concentrique à la surface du socle 15 et sont répartis sur un cercle dont le centre est disposé sur une droite passant par l'axe central de la cuve 2 à l'état posé de la cuve 2 sur le socle 15. Comme chaque capteur de poids est à la lecture indépendante, il est possible de vérifier la planéité du fond de la cuve à vide.

La cuve 2 est une cuve isolée thermiquement de l'extérieur, c'est-à-dire dont la paroi permet d'isoler le contenu de la cuve de l'extérieur pour éviter que le contenu de la cuve soit soumis à des variations de température résultant d'un transfert de chaleur entre l'atmosphère ambiante et le contenu de la cuve par passage à travers ladite paroi de la cuve. Cette cuve présente un corps de cuve tubulaire comportant une double paroi 3, 4 divisée en au moins deux zones décalées le long de l'axe longitudinal de la cuve. Chaque zone, qui délimite un volume annulaire régulable en température à l'aide de moyens de chauffage et de refroidissement, forme un échangeur thermique apte à refroidir ou à réchauffer le contenu de la cuve. Chaque échangeur 5, 6 est isolé thermiquement du ou des autres échangeur(s) thermique(s). Dans les exemples représentés, le corps de cuve comporte deux échangeurs thermiques superposés séparés l'un de l'autre par un joint annulaire. Ces échangeurs thermiques sont maintenus à l'état superposé par des brides, telles que des genouillères. En effet, au moins l'un 5 des échangeurs 5, 6 thermiques constitutifs du corps de cuve, dit échangeur 5 supérieur, se présente sous forme d'une pièce du type couronne séparable du reste du corps de cuve pour la réalisation d'une cuve de hauteur ajustable. Le corps de cuve tubulaire est fermé à une de ses extrémités par le fond 7 de la cuve et généralement ouvert à son autre extrémité. Cette extrémité ouverte forme l'ouverture d'introduction de l'échantillon dans la cuve. Cette ouverture peut être fermée si nécessaire à l'aide d'un couvercle. Le fond 7 de la cuve à double paroi délimite entre lesdites parois un volume régulable en température à l'aide de moyens de chauffage et de refroidissement dudit volume. Ce fond 7 forme un échangeur 8 thermique supplémentaire apte à réchauffer ou à refroidir le contenu de la cuve 2.

Il doit être noté qu'on entend par échangeur thermique, dans ce qui précède, comme dans tout ce qui suit, un appareil qui permet, à l'aide d'un fluide circulant à l'intérieur dudit appareil, de refroidir ou réchauffer un solide ou fluide au contact dudit appareil, en l'occurrence au contact de la paroi intérieure ou interne de ladite cuve. En effet, la cuve comporte, au niveau du corps de cuve, une paroi 3 périphérique externe isolée thermiquement de l'extérieur à l'aide d'un revêtement isolant, tel qu'une mousse de polyuréthane et une paroi 4 périphérique interne au contact de l'échantillon. Il en est de même pour le fond 7 de la cuve qui comporte également une paroi externe isolée thermiquement de l'extérieur et une paroi interne au contact de l'échantillon. Le fluide régulé en température qui circule entre lesdites parois externe et interne permet donc de réchauffer ou de refroidir l'échantillon contenu dans la cuve et en contact avec les parois intérieures de ladite cuve.

Pour permettre un tel fonctionnement de chaque échangeur thermique, chaque échangeur 5, 6, 8 thermique est muni de moyens 12, 13, 14 de raccordement à un réseau d'alimentation en fluide régulable en température, ce fluide formant les moyens de chauffage et de refroidissement dudit échangeur, lesdits moyens 12, 13, 14 de raccordement étant formés au moins par une entrée et une sortie de fluide à raccords rapides ménagées dans la paroi périphérique externe isolée thermiquement de la cuve à double paroi.

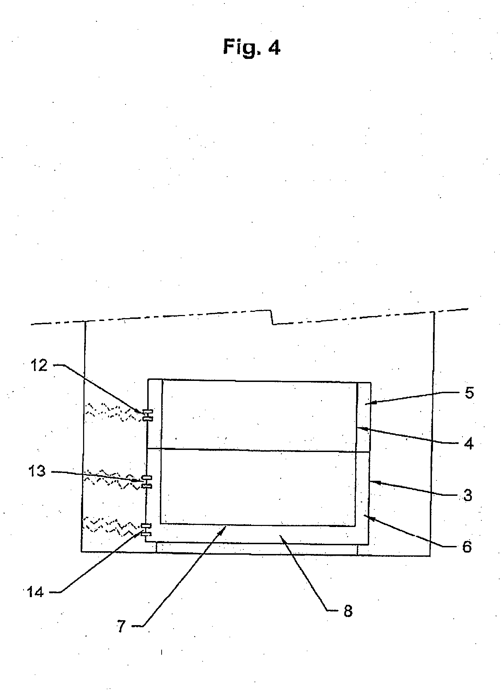

Ainsi, comme l'illustre la figure 4, chaque échangeur comporte sa propre entrée et sa propre sortie de fluide. Le réseau d'alimentation en fluide peut quant à lui comporter une installation de production et de stockage de fluide chaud et de fluide froid. Les deux fluides sont stockés chacun dans un ballon, dit l'un ballon de fluide chaud, l'autre ballon de fluide froid. Sur le circuit de circulation de fluide entre les ballons et les échangeurs thermiques, il est prévu des collecteurs intermédiaires, un jeu de vannes et des liaisons desdits circuits entre eux pour l'obtention de trois flux régulés en température qui alimentent chacun un échangeur thermique. Le pilotage des vannes s'opère à partir du système 20 électronique et/ou informatique de commande qui comporte des moyens de pilotage des moyens de chauffage et de refroidissement en fonction d'instructions introduites manuellement ou automatiquement dans ledit système 20 électronique et/ou informatique de commande.

Ce système 20 électronique et/ou informatique de commande comporte de manière classique des moyens de mémorisation d'information, des moyens d'analyse des informations mémorisées et des moyens de pilotage en fonction du résultat de l'analyse.

Dans les exemples représentés, le socle 15 est équipé de moyens 19 de guidage et de centrage de la cuve 2 lors du positionnement de la cuve 2 sur ledit socle 15. De préférence, les moyens 19 de guidage et de centrage sont formés de tiges en saillie de la surface du socle 15 servant à la réception de la cuve 2, lesdites tiges étant réparties sur un cercle à la surface du socle et délimitant chacune, à la manière d'un entonnoir depuis un point haut de la tige vers un point bas de la tige, une surface d'appui inclinée en direction du centre du cercle. Ainsi, lorsque la cuve a été positionnée au-dessus du socle, elle est automatiquement guidée et centrée par rapport audit socle à l'aide desdites tiges qui forment autour de la cuve une paroi inclinée à allure convergente en direction du centre dudit socle.

Les capteurs 16, 17, 18 de poids, en disposition concentrique à la surface du socle 15, sont répartis sur un cercle dont le centre est disposé sur une droite passant par l'axe central de la cuve 2 à l'état posé de la cuve 2 sur le socle 15. Ces capteurs de poids sont généralement formés par des jauges de contrainte.

Les informations issues des capteurs 16, 17, 18 de poids sont stockées à l'intérieur du système 20 électronique et/ou informatique de commande. Ces informations issues des capteurs 16, 17, 18 de poids permettent d'observer les migrations s'opérant au sein de l'échantillon, voire des changements de phase ou autre à l'intérieur dudit échantillon.

Comme mentionné ci-dessus, la cuve 2 étant montée amovible sur le socle 15, le fond 7 de la cuve 2 est muni d'une embase 9 équipée de passages 10 de fourche pour la manutention de la cuve 2. La cuve est ainsi aisément manipulable, ce qui facilite la prise d'échantillon sur site. Ladite embase 9 est équipée de pieds ou semelles 11 d'appui au sol de la cuve, lesdits pieds ou semelles 11 étant disposés à l'aplomb des capteurs 16, 17, 18 de poids à l'état posé de la cuve 2 sur son socle 15.

Dans une application particulière de l'invention, le réceptacle est destiné à être logé de manière amovible dans l'enceinte d'une cellule d'étude de l'environnement climatique. Ainsi, il est possible de faire varier en parallèle les conditions environnementales à l'intérieur de l'enceinte par action sur l'atmosphère de ladite enceinte et le différentiel thermique auquel l'échantillon est soumis.

Dans ces conditions, il est possible d'observer les migrations au sein dudit échantillon. Il en résulte un réceptacle particulièrement performant pour l'étude d'écosystème aquatique ou terrestre.

## Revendications

1. Réceptacle (1) pour la réception d'échantillon biologique,
**caractérisé en ce que** ledit réceptacle (1) est formé d'une cuve (2) cylindrique de réception d'échantillon isolée thermiquement de l'extérieur et d'un socle (15) sur lequel la cuve (2) est, en position dressée dite verticale, apte à reposer, ledit socle (15) étant équipé d'au moins trois capteurs (16, 17, 18) de poids à lecture indépendante, lesdits capteurs (16, 17, 18) de poids répartis à la surface du socle (15) étant sollicités en position d'appui de la cuve (2) sur le socle (15), cette cuve (2) présentant un corps de cuve comportant une double paroi (3, 4) divisée en au moins deux zones décalées le long de l'axe longitudinal de la cuve, chaque zone, qui délimite un volume annulaire régulable en température à l'aide de moyens de chauffage et de refroidissement, formant un échangeur thermique apte à refroidir ou à réchauffer le contenu de la cuve, chaque échangeur (5, 6) étant isolé thermiquement du ou des autres échangeur(s) thermique(s).

2. Réceptacle (1) selon la revendication 1,
**caractérisé en ce que** le socle (15) est équipé de moyens (19) de guidage et de centrage de la cuve (2) lors du positionnement de la cuve (2) sur ledit socle (15).

3. Réceptacle (1) selon la revendication 2,
**caractérisé en ce que** les moyens (19) de guidage et de centrage sont formés de tiges en saillie de la surface du socle (15) servant à la réception de la cuve (2), lesdites tiges étant réparties sur un cercle à la surface du socle et délimitant chacune, à la manière d'un entonnoir depuis un point haut de la tige vers un point bas de la tige, une surface d'appui inclinée en direction du centre du cercle.

4. Réceptacle (1) selon l'une des revendications 1 à 3,
**caractérisé en ce que** les capteurs (16, 17, 18) de poids, en disposition concentrique à la surface du socle (15), sont répartis sur un cercle dont le centre est disposé sur une droite passant par l'axe central de la cuve (2) à l'état posé de la cuve (2) sur le socle (15).

5. Réceptacle (1) selon l'une des revendications 1 à 4,
**caractérisé en ce que** au moins l'un (5) des échangeurs (5, 6) thermiques constitutifs du corps de cuve, dit échangeur (5) supérieur, se présente sous forme d'une pièce du type couronne séparable du reste du corps de cuve pour la réalisation d'une cuve de hauteur ajustable.

6. Réceptacle (1) selon l'une des revendications 1 à 5,
**caractérisé en ce que** la cuve (2) présente un fond (7) à double parois délimitant entre lesdites parois un volume régulable en température à l'aide de moyens de chauffage et de refroidissement dudit volume, ce fond (7) formant un échangeur (8) thermique supplémentaire apte à réchauffer ou a refroidir le contenu de la cuve (2).

7. Réceptacle (1) selon l'une des revendications 1 à 6,
**caractérisé en ce que** la cuve (2) étant montée amovible sur le socle (15), le fond (7) de la cuve (2) est muni d'une embase (9) équipée de passages (10) de fourche pour la manutention de la cuve (2).

8. Réceptacle (1) selon la revendication 7,
**caractérisé en ce que** ladite embase (9) est équipée de pieds ou semelles (11) d'appui au sol de la cuve, lesdits pieds ou semelles (11) étant disposés à l'aplomb des capteurs (16, 17, 18) de poids à l'état posé de la cuve (2) sur son socle (15).

9. Réceptacle (1) selon l'une des revendications 1 à 8,
**caractérisé en ce que** chaque échangeur (5, 6, 8) thermique est muni de moyens (12, 13, 14) de raccordement à un réseau d'alimentation en fluide régulable en température, ce fluide formant les moyens de chauffage et de refroidissement dudit échangeur, lesdits moyens (12, 13, 14) de raccordement étant formés au moins par une entrée et une sortie de fluide à raccords rapides ménagées dans la paroi périphérique externe isolée thermiquement de la cuve à double paroi.

10. Réceptacle (1) selon l'une des revendications 1 à 9,
**caractérisé en ce que** les informations issues des capteurs (16, 17, 18) de poids sont stockées à l'intérieur d'un système (20) électronique et/ou informatique de commande, comportant des moyens de pilotage des moyens de chauffage et de refroidissement des échangeurs thermiques.

11. Cellule d'étude de l'environnement climatique apte à recevoir un échantillon biologique à étudier, ladite cellule comprenant au moins :
- une enceinte,
- des moyens de génération de l'environnement climatique aptes à modifier l'état de l'atmosphère intérieur de l'enceinte de ladite cellule, cet état se caractérisant au moins par les paramètres température, humidité, ensoleillement, pression, vent, précipitations, teneur en gaz et
- un réceptacle (1) logé à l'intérieur de l'enceinte de ladite cellule,
**caractérisée en ce que** le réceptacle (1) servant à la réception de l'échantillon est conforme à l'une des revendications 1 à 10.

## Patentansprüche

1. Behälter (1) zur Aufnahme einer biologischen Probe,
**dadurch gekennzeichnet, dass** der Behälter (1) von einem zylindrischen, thermisch gegenüber der Außenwelt isolierten Probenaufnahmetank (2) und von einem Sockel (15), auf dem der Tank (2) in vertikaler aufgerichteter Position zu ruhen imstande ist, gebildet ist, wobei der Sockel (15) mit mindestens drei Gewichtssensoren (16, 17, 18) mit unabhängiger Ablesung ausgestattet ist, wobei die auf der Oberfläche des Sockels (15) verteilten Gewichtssensoren (16, 17, 18) in Abstützposition des Tanks (2) auf dem Sockel (15) beansprucht werden, wobei dieser Tank (2) einen Tankkörper aufweist, der eine doppelte Wand (3, 4) aufweist, die in mindestens zwei Zonen unterteilt ist, die entlang der Längsachse des Tanks versetzt sind, wobei jede Zone, die ein mit Hilfe von Heiz- und Kühlmitteln temperaturregulierbares ringförmiges Volumen begrenzt, einen Wärmetauscher bildet, der imstande ist, den Inhalt des Tanks zu kühlen oder zu erwärmen, wobei jeder Tauscher (5, 6) von dem oder den anderen Wärmetauscher(n) thermisch isoliert ist.

2. Behälter (1) nach Anspruch 1,
**dadurch gekennzeichnet, dass** der Sockel (15) mit Führungs- und Zentriermitteln (19) des Tanks (2) bei der Positionierung des Tanks (2) auf dem Sockel (15) ausgestattet ist.

3. Behälter (1) nach Anspruch 2,
**dadurch gekennzeichnet, dass** die Führungs- und Zentriermittel (19) von Stangen gebildet sind, die aus der Oberfläche des Sockels (15) hervorstehen, die dem Empfang des Tanks (2) dienen, wobei die Stangen auf einem Kreis auf der Oberfläche des Sockels verteilt sind und jeweils in der Art eines Trichters von einem oberen Punkt der Stange zu einem unteren Punkt der Stange eine in Richtung des Zentrums des Kreises geneigte Stützfläche begrenzen.

4. Behälter (1) nach einem der Ansprüche 1 bis 3,
**dadurch gekennzeichnet, dass** die Gewichtssensoren (16, 17, 18) in konzentrischer Anordnung auf der Oberfläche des Sockels (15) auf einem Kreis verteilt sind, dessen Zentrum auf einer Geraden angeordnet ist, die im aufgesetzten Zustand des Tanks (2) auf dem Sockel (15) durch die zentrale Achse des Tanks (2) verläuft.

5. Behälter (1) nach einem der Ansprüche 1 bis 4,
**dadurch gekennzeichnet, dass** mindestens einer (5) der strukturellen Wärmetauscher (5, 6) des Tankkörpers, als oberer Tauscher (5) bezeichnet, in Form eines Teils vom Typ vom restlichen Tankkörper trennbarer Kranz für die Realisierung eines Tanks regulierbarer Höhe ist.

6. Behälter (1) nach einem der Ansprüche 1 bis 5,
**dadurch gekennzeichnet, dass** der Tank (2) einen doppelwandigen Boden (7) aufweist, der zwischen den Wänden ein mit Hilfe von Heiz- und Kühlmitteln des Volumens temperaturregulierbares Volumen begrenzt, wobei dieser Boden (7), der einen zusätzlichen Wärmetauscher (8) bildet, imstande ist, den Inhalt des Tanks (2) zu erwärmen oder zu kühlen.

7. Behälter (1) nach einem der Ansprüche 1 bis 6,
**dadurch gekennzeichnet, dass** der Tank (2) lösbar auf dem Sockel (15) montiert ist, wobei der Boden (7) des Tanks (2) mit einer Basis (9) ausgestattet ist, die mit Gabeldurchgängen (10) für den Transport des Tanks (2) ausgestattet ist.

8. Behälter (1) nach Anspruch 7,
**dadurch gekennzeichnet, dass** die Basis (9) mit Stützfüßen oder - sohlen (11) auf dem Boden des Tanks ausgestattet ist, wobei die Füße oder Sohlen (11) im aufgesetzten Zustand des Tanks (2) auf dem Sockel (15) lotrecht zu den Gewichtssensoren (16, 17, 18) angeordnet sind.

9. Behälter (1) nach einem der Ansprüche 1 bis 8,
**dadurch gekennzeichnet, dass** jeder Wärmetauscher (5, 6, 8) mit Anschlussmitteln (12, 13, 14) an ein temperaturregulierbares Fluidversorgungsnetz ausgestattet ist, wobei dieses Fluid die Heiz- und Kühlmittel des Tauschers bildet, wobei die Anschlussmittel (12, 13, 14) von mindestens einem Fluideingang und -ausgang mit Schnellanschlüssen gebildet ist, die in der thermisch isolierten äußeren Umfangswand des doppelwandigen Tanks ausgebildet sind.

10. Behälter (1) nach einem der Ansprüche 1 bis 9,
**dadurch gekennzeichnet, dass** die von den Gewichtssensoren (16, 17, 18) ausgehenden Informationen im Innern eines elektronischen und/oder EDV-Steuersystems (20) gespeichert werden, das Steuermittel der Heiz- und Kühlmittel der Wärmetauscher aufweist.

11. Zelle für die Untersuchung der klimatischen Umgebung, die imstande ist, eine zu untersuchende biologischen Probe aufzunehmen, wobei die Zelle mindestens umfasst:
- einen Mantel,
- Mittel für die Erzeugung der klimatischen Umgebung, die imstande sind, den Zustand der inneren Atmosphäre des Mantels der Zelle zu verändern, wobei dieser Zustand mindestens durch die Parameter Temperatur, Feuchtigkeit, Sonneneinstrahlung, Druck, Wind, Niederschläge, Gasgehalt gekennzeichnet ist, und
- einen im Innern des Mantels untergebrachten Behälter (1),
**dadurch gekennzeichnet, dass** der zur Aufnahme der Probe dienende Behälter (1) einem der Ansprüche 1 bis 10 entspricht.

## Claims

1. A container (1) for receiving a biological sample,
**characterized in that** said container (1) is formed by a cylindrical tank (2) for receiving a sample thermally isolated from the outside and a base (15) on which the tank (2) is, in the so-called vertical upright position, able to rest, said base (15) being equipped with at least three weight sensors (16, 17, 18) with independent reading, said weight sensors (16, 17, 18) distributed on the surface of the base (15) being biased in the position bearing of the tank (2) on the base (15), said tank (2) having a tank body including a double wall (3, 4) divided into at least two zones offset along the longitudinal axis of the tank, each zone, which delimits an annular volume with an adjustable temperature using heating and cooling means, forming a heat exchanger able to cool or heat the content of the tank, each exchanger (5, 6) being thermally isolated from the other heat exchanger(s).

2. The container (1) according to claim 1,
**characterized in that** the base (15) is equipped with means (19) for guiding and centering the tank (2) during positioning of the tank (2) on said base (15).

3. The container (1) according to claim 2,
**characterized in that** the guiding and centering means (19) are formed from rods protruding from the surface of the base (15) serving to receive the tank (2), said rods being distributed on a circle at the surface of the base and each delimiting, like a funnel from a high point of the rod toward a low point of the rod, a bearing surface inclined toward the center of the circle.

4. The container (1) according to one of claims 1 to 3,
**characterized in that** the weight sensors (16, 17, 18), in a concentric arrangement on the surface of the base (15), are distributed on a circle whereof the center is positioned on a straight line passing through the central axis of the tank (2) in the state with the tank (2) placed on the base (15).

5. The container (1) according to one of claims 1 to 4,
**characterized in that** at least one (5) of the heat exchangers (5, 6) making up the tank body, called upper exchanger (5), assumes the form of a crown-type piece separable from the rest of the tank body to produce a tank with an adjustable height.

6. The container (1) according to one of claims 1 to 5,
**characterized in that** the tank (2) has a bottom (7) with double walls delimiting, between said walls, a volume with a temperature adjustable using heating and cooling means for said volume, that bottom (7) forming an additional heat exchanger (8) able to heat or cool the content of the tank (2).

7. The container (1) according to one of claims 1 to 6,
**characterized in that** the tank (2) being removably mounted on the base (15), the bottom (7) of the tank (2) is provided with a base (9) equipped with fork passages (10) for handling the tank (2).

8. The container (1) according to claim 7,
**characterized in that** said base (9) is equipped with feet or base plates (11) for bearing on the floor of the tank, said feet or base plates (11) being positioned overhanging the weight sensors (16, 17, 18) in the state with the tank (2) placed on its base (15).

9. The container (1) according to one of claims 1 to 8,
**characterized in that** each heat exchanger (5, 6, 8) is provided with means (12, 13, 14) for connecting to a fluid supply network with an adjustable temperature, that fluid forming the heating and cooling means for said exchanger, said connecting means (12, 13, 14) being formed at least by a fluid inlet and outlet with quick couplers arranged in the thermally insulated outer peripheral wall of the double-walled tank.

10. The container (1) according to one of claims 1 to 9,
**characterized in that** the information from the weight sensors (16, 17, 18) is stored within an electronic and/or computerized control system (20), including control means for the heating and cooling means of the heat exchangers.

11. A climatic environment research cell able to receive a biological sample to be studied, said cell comprising at least:
- an enclosure,
- means for generating the climatic environment able to modify the state of the atmosphere inside the enclosure of said cell, that state being characterized at least by the temperature, humidity, sunshine, pressure, wind, precipitation, gas content parameters, and
- a container (1) housed inside the enclosure of said cell,
**characterized in that** the container (1) used to receive the sample is according to one of claims 1 to 10.
